# EUROPÄISCHE PATENTSCHRIFT

(11) **EP 0 542 242 B1**
(45) Veröffentlichungstag und Bekanntmachung des Hinweises auf die Patenterteilung: **04.06.1997**
(21) Anmeldenummer: 92119310.8
(22) Anmeldetag: 11.11.1992
(51) Int. Cl.: A61F 2/52, C09J 7/02, C09J 175/04

(54) **Verfahren zum Herstellen einer Brustprothese**
Process for manufacturing a mammary prosthesis
Procédé de fabrication de prothèse mammaire

(30) Priorität: 14.11.1991 DE 9114201 U; 21.11.1991 DE 9114512 U; 06.04.1992 DE 4211542
(43) Veröffentlichungstag der Anmeldung: 19.05.1993
(73) Patentinhaber: AMOENA Medizin-Orthopädie-Technik GmbH, D-83064 Raubling (DE)
(72) Erfinder: Wild, Helmut Franz, W-8201 Rohrdorf (DE)
(74) Vertreter: Laufhütte, Dieter, Dr.-Ing.

(56) Entgegenhaltungen:
- EP-A- 0 392 960
- EP-A- 0 433 636
- DE-A- 2 701 627
- DE-A- 2 742 394
- DE-A- 2 802 375
- DE-U- 9 010 426
- GB-A- 2 202 745

## Beschreibung

Die Erfindung betrifft ein verfahren zur Herstellung einer Brustprothese nach dem Oberbegriff des Anspruchs 1.

Zur Herstellung von Brustprothesen ist es bereits bekannt, daß eine weich-elastisch eingestellte additionsvernetzende Zwei-Komponenten-Silikon-Kautschuk-Masse in eine die Außenseite und Innenseite der Prothese bildende Kunststoffolie eingefüllt wird. Die mit der noch nicht vernetzten Silikon-Kautschuk-Masse gefüllte Kunststoffolie wird in eine über einen Deckel verschließbare Form eingelegt. Die verschlossene Form wird mit der noch nicht vernetzten Silikon-Kautschuk-Masse in einen Ofen gestellt, so daß die Silikon-Kautschuk-Masse unter Wärmeeinwirkung vernetzt werden kann.

Ein besonderes Problem bei Brustprothesen, die beispielsweise mit diesem Verfahren hergestellt sind, ist es, diese gut und möglichst unverrutschbar an der Brust der Trägerin zu befestigen.

Bei einer aus dem DE-GM 90 10 426 bekannten Brustprothese dieser Art erfolgt die Befestigung dadurch, daß die Prothesenkörper in die Körbchen eines Büstenhalters eingelegt werden, die zusätzlich zur Halterung der Prothesenkörper mit Einlegetaschen versehen sein können.

Eine günstige Halterung einer Brustprothese ist dadurch erreichbar, wenn diese unmittelbar auf der Haut bzw. dem Narbenbereich der Trägerin befestigt wird.

Eine aus der EP-A-392 960 bekannte Brustprothese der eingangs angegebenen Art ist innerhalb eines umlaufenden lippenförmigen Randes auf ihrer Rückseite mit einer umlaufenden, durch einen Absatz gebildeten Schulter versehen, auf der Haftstreifen oder Haftstücke befestigt sind, die mit den Haftbereichen von auf dem Körper der Frau durch hautfreundliche Klebemittel befestigte Streifen in der Weise zusammenwirken, daß die Prothese mit den auf der Haut klebenden Haltestreifen verbunden ist und von diesen wieder gelöst werden kann. Als bevorzugtes Befestigungsmittel ist dabei eine Klettverbindung vorgesehen. Bei diesen vorbekannten Brustprothesen werden die Haltestreifen oder Haftstücke auf die sonst fertige Brustprothese mittels eines konventionellen Klebers aufgeklebt. Dabei hat es sich als schwierig und aufwendig erwiesen, die entsprechenden Haftstreifen oder Haftstücke genau zu positionieren. Eine genaue Positionierung ist aber notwendig, damit die Haftstücke mit den auf der Haut der Trägerin mittels einer Schablone aufgebrachten Haftstücke in Überdeckung gebracht werden können. Andererseits ergibt sich durch das nachträgliche Aufkleben mit lösungsmittelhaltigen Klebern, wie dies beispielsweise aus der DE 28 02 375 A bekannt ist, eine unbefriedigende Verklebung, die durch Wellenbildungen, teilweise Ablösungen oder unsaubere Randbereiche der aufgeklebten Haftstreifen oder Haftstücke zutage treten.

Aus der EP-A-433 636 ist ein Verfahren zur Herstellung von Brustprothesen bekannt, bei der eine additionsvernetzende Silikon-Masse in eine die Außenseite und Innenseite der Prothese bildende Kunststoffolie eingefüllt wird, wobei die mit der noch nicht vernetzten Silikon-Masse gefüllte Kunststoffolie in eine Form eingelegt - und die Silikon-Masse unter Wärmeeinwirkung vernetzt wird.

Aufgabe der Erfindung ist es daher, das bereits bekannte Verfahren zur Herstellung von Brustprothesen derart weiterzubilden, daß die aufzubringenden Halteelemente einfach auf die Brustprothese aufgebracht werden können und daß die fertige Brustprothese ein sauberes Erscheinungsbild aufweist.

Erfindungsgemäß wird diese Aufgabe, ausgehend von dem gattungsgemäßen Verfahren, dadurch gelöst, daß mindestens ein auf die die Innenseite der Brustprothese bildende Kunststoffolie durch Aktivierung eines wärmeaushärtenden Klebers (Hot-Melt-Kleber) aufzuklebendes Halteelement, vorzugsweise ein Haltestreifen, auf das die Klebeschicht aufgetragen ist, mittels eines entsprechenden Gegenstückes auf dem Formendeckel positioniert wird und daß dieses nach Schließen des Formendeckels an der gewünschten Position auf der Innenseite der Brustprothese positioniert ist, daß durch die Wärmeeinwirkung während der Vernetzung der Silikon-Kautschuk-Masse die Klebeschicht aktiviert wird und daß das Halteelement nach Beendigung der Vernetzung das Öffnen des Formendeckels von dem Gegenstück am Formendeckel gelöst wird.

Das mindestens eine Halteelement besteht vorteilhaft aus einem Teil eines zweiteiligen Klettbandes.

Vorzugsweise enthalten sowohl die eingesetzte Kunststoffolie als auch der wärmehärtende Kleber Polyurethan.

Als Halteelement kann eine auf einer Tragfolie aufgebrachte, dauernd klebrig bleibende Schicht eingesetzt werden. Derartig dauernd klebrig bleibende Schichten auf Kunststoffbasis sind an sich bekannt. Diese Schichten haben den Vorteil, daß sie eine Verklebung der Brustprothese mit der Haut der Trägerin ermöglichen, ohne daß nach dem Abnehmen der Prothese Reste der klebrigen Schicht auf der Haut der Trägerin verbleiben.

Nach einer besonders vorteilhaften Ausgestaltung ist es vorgesehen, daß die dauernd klebrig bleibende Schicht auf einer Tragfolie aufgebracht ist, deren Rückseite durch eine lösbare Haftverbindung mit der komplementären Haftschicht einer mit der Prothese verklebten Verbindungsfolie verbindbar ist. Hat die Dauerklebeschicht nach längerem Gebrauch ihre Klebrigkeit verloren, kann sie zusammen mit ihrer Tragfolie abgezogen und durch eine neue, eine frische Dauerklebschicht tragende Tragfolie ersetzt werden.

Das Haftelement kann nahezu die gesamte Innenseite der Brustprothese überdecken. Dies ist besonders dann vorteilhaft, wenn verhältnismäßig schwere Brustprothesen zu halten sind.

Alternativ dazu kann sich das Haftelement über einen Randbereich der Innenseite der Brustprothese erstrecken. Es können auch mehrere Haftelemente über den Randbereich oder die gesamte Fläche verteilt auf die Innenseite der Brustprothese geklebt werden.

## Patentansprüche

1. Verfahren zur Herstellung einer Brustprothese mit folgenden Schritten:
- eine weich-elastisch eingestellte additionsvernetzende Zwei-Komponenten-Silikon-Kautschuk-Masse wird in eine die Außenseite und Innenseite der Prothese bildende Kunststoffolie eingefüllt,
- die mit der nocht nicht vernetzten Silikon-Kautschuk-Masse gefüllte Kunststoffolie wird in eine Form eingelegt und
- die Silikon-Kautschuk-Masse wird unter Wärmeeinwirkung vernetzt,
**dadurch gekennzeichnet**,
daß mindestens ein auf die die Innenseite der Brustprothese bildende Kunststoffolie durch Aktivierung eines wärmeaushärtenden Klebers aufzuklebendes Halteelement, vorzugsweise ein Haftstreifen, auf das die Klebeschicht aufgetragen ist, mittels eines entsprechenden Gegenstückes auf dem Formendeckel positioniert wird und daß dieses nach Schließen des Formendeckels an der gewünschten Position auf der Innenseite der Brustprothese positioniert ist, daß durch die Wärmeeinwirkung während der Vernetzung der Silikon-Kautschuk-Masse die Klebeschicht aktiviert wird und daß das Halteelement nach Beendigung der Vernetzung durch das Öffnen des Formendeckels von dem Gegenstück am Formdeckel gelöst wird.

2. Verfahren nach Anspruch 1, dadurch gekennzeichnet, daß das mindestens eine Halteelement aus einem Teil eines zweiteiligen Klettbandes besteht.

3. Verfahren nach Anspruch 1 oder 2, dadurch gekennzeichnet, daß sowohl die eingesetzte Kunststoffolie als auch der wärmehärtende Kleber Polyurethan enthalten.

4. Verfahren nach einem der Ansprüche 1 bis 3, dadurch gekennzeichnet, daß das mindestens eine Halteelement eine auf eine Tragfolie aufgebrachte, dauernd klebrig bleibende Schicht ist.

5. Verfahren nach einem der Ansprüche 1 bis 4, dadurch gekennzeichnet, daß als Halteelement eine Haftschicht eingesetzt wird, die mit einer komplementären Tragfolie, auf die die dauernd klebrig bleibende Schicht aufgebracht ist, verbindbar ist.

6. Verfahren nach einem der Ansprüche 1 bis 5, dadurch gekennzeichnet, daS das Haftelement großflächig die Innenseite der Brustprothese bedeckt.

7. Verfahren nach einem der Ansprüche 1 bis 5, dadurch gekennzeichnet, daß das Haftelement einen Randbereich der Innenseite der Brustprothese überdeckt.

8. Verfahren nach einem der Ansprüche 1 bis 5, dadurch gekennzeichnet, daß mehrere Haftelernent über einen Randbereich oder die gesamte Fläche der Innenseite der Brustprothese verteilt aufgeklebt werden.

## Claims

1. Process for producing a breast prosthesis, comprising the following steps:
- an addition-crosslinking two-component silicone rubber composition which has been formulated to be flexible is used to fill a plastics film which forms the outside and inside of the prosthesis,
- the plastics film filled with the as yet uncrosslinked silicone rubber composition is placed in a mould, and
- the silicone rubber composition is crosslinked under the action of heat,
characterized
in that at least one retaining element, which is to be stuck onto the plastics film forming the inside of the breast prosthesis by the activation of a heat-curing adhesive and is preferably an adhesive strip, onto which element the adhesive layer is applied, is positioned on the mould lid by means of a corresponding counterpart, and in that this element, following the closure of the mould lid, is positioned at the desired position on the inside of the breast prosthesis, in that the adhesive layer is activated by the action of heat during the crosslinking of the silicone rubber composition, and in that, after the end of crosslinking, the retaining element is detached from the counterpart on the mould lid as a result of opening the mould lid.

2. Process according to Claim 1, characterized in that the retaining element of which there is at least one consists of one part of a two-part touch-and-close tape.

3. Process according to Claim 1 or 2, characterized in that both the plastics film employed and the heat-curing adhesive comprise polyurethane.

4. Process according to one of Claims 1 to 3, characterized in that the retaining element of which there is at least one is a layer which is applied to a carrier film and which remains permanently tacky.

5. Process according to one of Claims 1 to 4, characterized in that the retaining element employed is an adhesion layer which can be connected to a complementary carrier film to which the permanently tacky layer is applied.

6. Process according to one of Claims 1 to 5, characterized in that the adhesion element covers a large area of the inside of the breast prosthesis.

7. Process according to one of Claims 1 to 5, characterized in that the adhesion element covers an edge region of the inside of the breast prosthesis.

8. Process according to one of Claims 1 to 5, characterized in that a plurality of adhesion elements are bonded in distribution over an edge region or the entire area of the inside of the breast prosthesis.

## Revendications

1. Procédé de fabrication d'une prothèse mammaire comprenant les étapes consistants à :
- une masse en caoutchouc silicone à deux composants additionnés par réticulation, souple et élastique est introduite dans une feuille de matière synthétique formant le côté extérieur et le côté intérieur de la prothèse,
- la feuille en matière synthétique remplie de la masse en caoutchouc silicone non encore réticulée est placée dans un moule et
- la masse en caoutchouc silicone est réticulée sous l'action de la chaleur,
caractérisé en ce qu'au moins un élément de retenue, de préférence une bande de retenue, sur lequel est appliquée la couche de colle, à coller sur la feuille en matière synthétique formant le côté intérieur de la prothèse mammaire, par activation d'une colle thermodurcissable, est positionné au moyen d'une contre-pièce correspondante sur le couvercle de moule et qu'il est positionné, après la fermeture du couvercle de moule, à la position voulue au côté intérieur de la prothèse mammaire, en ce que, sous l'action de la chaleur, pendant la réticulation de la masse en caoutchouc silicone, la couche de colle est activée et en ce que l'élément de retenue, après avoir terminé la réticulation, est détaché en ouvrant le couvercle de moule de la contre-pièce au couvercle de moule.

2. Procédé selon la revendication 1, caractérisé en ce que l'élément de retenue, dont au moins un est prévu, est constitué d'une partie d'une bande auto-accrochante en deux parties.

3. Procédé selon la revendication 1 ou 2, caractérisé en ce que, aussi bien la feuille en matière synthétique utilisée, que la colle thermodurcissable contiennent du polyuréthane.

4. Procédé selon l'une des revendications 1 à 3, caractérisé en ce que l'élément de retenue précité est une couche qui reste collante en permanence et qui est appliquée sur une feuille de support.

5. Procédé selon l'une des revendications 1 à 4, caractérisé en ce qu'il est utilisé comme élément de retenue une couche d'adhésion qui peut être assemblée avec une feuille de support complémentaire sur laquelle est appliquée la couche qui reste collante en permanence.

6. Procédé selon l'une des revendications 1 à 5, caractérisé en ce que l'élément d'adhésion couvre une grande partie de la surface du côté intérieur de la prothèse mammaire.

7. Procédé selon l'une des revendications 1 à 5, caractérisé en ce que l'élément d'adhésion couvre une zone de bord du côté intérieur de la prothèse mammaire.

8. Procédé selon l'une des revendications 1 à 5, caractérisé en ce que plusieurs éléments d'adhésion sont collés sur une zone de bord ou sont répartis sur toute la surface du côté intérieur de la prothèse mammaire.
